(19) Europäisches Patentamt
European Patent Office
Office européen des brevets

(11) Veröffentlichungsnummer: **0 423 068 B1**

(12) **EUROPÄISCHE PATENTSCHRIFT**

(45) Veröffentlichungstag der Patentschrift: **30.11.94**

(51) Int. Cl.5: **C09B 1/00**, C08K 5/20, C08L 23/02, //C07C233/33, C07C235/56

(21) Anmeldenummer: **90810750.1**

(22) Anmeldetag: **02.10.90**

Die Akte enthält technische Angaben, die nach dem Eingang der Anmeldung eingereicht wurden und die nicht in dieser Patentschrift enthalten sind.

# (54) Neue Dianthrachinonylverbindungen.

(30) Priorität: **10.10.89 CH 3693/89**

(43) Veröffentlichungstag der Anmeldung: **17.04.91 Patentblatt 91/16**

(45) Bekanntmachung des Hinweises auf die Patenterteilung: **30.11.94 Patentblatt 94/48**

(84) Benannte Vertragsstaaten: **CH DE FR GB IT LI**

(56) Entgegenhaltungen:
**DE-A- 1 794 300**
**DE-A- 2 200 115**
**FR-A- 2 149 194**

**CHEMICAL ABSTRACTS, Band 81, Nr. 16, 21. Oktober 1974, Seiten 85-86,Zusammenfassung Nr. 93064j, Columbus, Ohio, US;& JP-A-74 01 289 (DAINIPPON INK AND CHEMICALS, INC.) 12-01-1974**

(73) Patentinhaber: **CIBA-GEIGY AG**
**Klybeckstrasse 141**
**CH-4002 Basel (CH)**

(72) Erfinder: **Wallquist, Olof, Dr.**
**Rte. du Confin 31**
**CH-1723 Marly (CH)**
Erfinder: **De Weck, Guy, Dr.**
**Totentanz 11**
**CH-4051 Basel (CH)**
Erfinder: **Wooden, Gary, Dr.**
**Gansmatt 351**
**CH-1716 Oberschrot (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 423 068 B1

## Beschreibung

Die vorliegende Erfindung betrifft die Verwendung von Dianthrachinonylverbindungen enthaltend bestimmte Carbonylaminogruppen mit höheren Alkylresten zum Massefärben von Kunststoffen, insbesondere von Polyolefinen.

Dianthrachinonylverbindungen enthaltend, ausser mindestens einen Halogenatom, einer Alkyl-oder Nitrogruppe, niedere Acylaminogruppen oder speziell substituierte Benzoylaminogruppen in 4- und 4'-Stellung und ihre Verwendung zum Pigmentieren von Anstrichstoffen, Kunstharzen, Kunststofffasern, Druckfarben, Gummi, u.s.w. sind im japanischen Patent 743346 beschrieben. Obwohl sie bereits gute Allgemeinbeständigkeiten aufweisen, genügen sie bei gewissen Applikationen nicht immer den heutigen hohen Anforderungen der Technik. FR-A 2149194 beschreibt 1,1'-Dianthrachinonylverbindungen, die in 2- und 2'-Stellung je eine Acetylaminogruppe aufweisen, als Zwischenprodukte für die Herstellung von Farbstoffen, insbesondere von Flavanthronen.

Es ist nun gefunden worden, dass durch die Einführung höherer Acylaminogruppen in 4- und 4'-Stellung von Dianthrachinonylverbindungen eine überraschende Verbesserung der Pigmenteigenschaften, insbesondere der Dispergierbarkeit, sowie der Licht-, Wetter- und Hitzebeständigkeit, erzielt werden kann.

Die vorliegende Erfindung betrifft demnach Kunststoffe enthaltend in der Masse Dianthrachinonylverbindungen der Formel

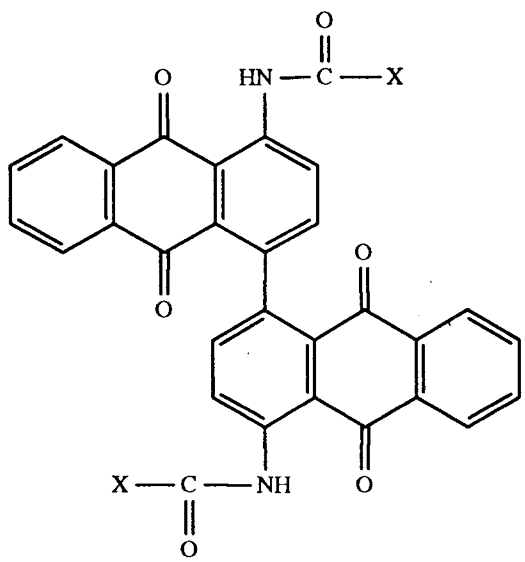

(I),

worin X einen Rest $R_1$, $R_2$, $R_3$ oder $R_4$ bedeutet, wobei
$R_1$ Alkyl oder Alkenyl mit mindestens 8 C-Atomen,
$R_2$ $-OR_1$ oder $-SR_1$,
$R_3$ $-NHR_1$ oder $-N(R_1)_2$ sind und
$R_4$ eine Gruppe der Formel

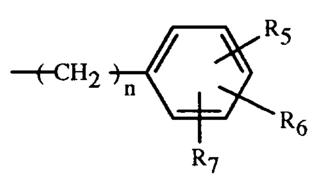

bedeutet, worin
$R_5$ $-NHR_1$, $-N(R_1)_2$, $-OR_1$ oder $-SR_1$ ist und n Null, 1 oder 2 bedeutet und
$R_6$ und $R_7$, unabhängig voneinander, Wasserstoff, Halogen, Nitro, $C_1$-$C_4$-Alkyl oder $C_1$-$C_4$-Alkoxy bedeuten.

$R_1$ als Alkyl mit mindestens 8 C-Atomen ist verzweigtes oder geradkettiges Alkyl, z.B. n-Octyl, n-Nonyl, n-Decyl, n-Dodecyl, n-Tridecyl, Isotridecyl, n-Tetradecyl (Myristyl), n-Pentadecyl, n-Hexadecyl, 1-Methylpentadecyl, n-Octadecyl, n-Eicosyl, n-Tetracosyl, n-Hexacosyl, n-Triacontyl und n-Pentacontyl.

$R_1$ als Alkenyl mit mindestens 8 C-Atomen ist verzweigtes oder geradkettiges Alkenyl, z.B. 8-Heptadecenyl oder 9-Decenyl.

$R_1$ steht bevorzugt für $C_{10}$- bis $C_{35}$-Alkyl, insbesondere für $C_{12}$- bis $C_{18}$-Alkyl, oder Gemische davon, und leitet sich z.B. von langkettigen Alkanolen, wie z.B. n-Decyl-, Lauryl-, Myristyl-, Cetyl- und Stearylalkohol, sowie von den als ®Alfole (Fa. Condea) erhältlichen, unverzweigten primären Alkoholen mit einer stets geraden Zahl von C-Atomen (die Alkylgruppen werden hier als "Alfyl" bezeichnet, z.B. Alfyl-$C_{12}$, Alfyl-$C_{14}$) ab, die gegebenenfalls als Ausgangsprodukte verwendet werden können.

Ganz besonders bevorzugt sind Kunststoffe enthaltend Dianthrachinonylverbindungen der Formel I, worin X $C_{12}$-$C_{18}$-Alkyl oder eine Gruppe der Formel

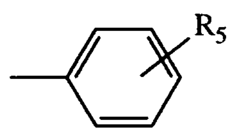

bedeutet,
worin $R_5$ -$NHR_1$, -$N(R_1)_2$, -$OR_1$ oder -$SR_1$ ist und $R_1$ $C_{12}$-$C_{18}$-Alkyl bedeutet und insbesondere solche, worin X geradkettiges $C_{12}$-$C_{18}$-Alkyl ist.

Die Verbindungen der Formel I können in Analogie zu an sich bekannten Verfahren hergestellt werden, z.B. ausgehend von einem Mol der Verbindung der Formel

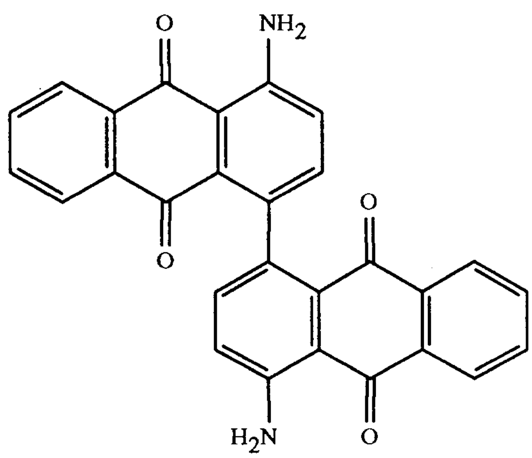

(II),

a) wenn X $R_1$, $OR_1$, $SR_1$, $N(R_1)_2$ oder $R_4$ ist,
durch Kondensation mit zwei Mol einer Verbindung der Formel

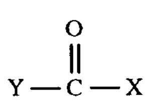

(III);

b) wenn X NH-$R_1$ ist,
durch Addition von zwei Mol einer Verbindung der Formel

$O=C=N-R_1$ (IV)

und

c) wenn X $OR_1$, $SR_1$, $NHR_1$ oder $N(R_1)_2$ ist,
durch Kondensation mit zwei Mol Phosgen zum Diisocyanat und anschliessende Addition von zwei Mol eines Alkohols der Formel

$HOR_1$, (V),

eines Amins der Formeln

$H_2NR_1$ (VI), oder $HN(R_1)_2$ (VII),

oder eines Merkaptans der Formel

$HSR_1$ (VIII),

wobei in der Formel III Y Halogen, z.B. Brom oder insbesondere Chlor ist, und in den Formeln IV bis VIII $R_1$ die oben angegebene Bedeutung hat.

Die Verbindungen II bis VIII sind bekannt. Sollten allerdings einige davon noch neu sein, so können sie nach an sich bekannten Methoden hergestellt werden.

Die Kondensations- bzw. Additionsverfahren gemäss a), b) und c) werden zweckmassig in Gegenwart eines organischen inerten Lösungsmittels bei Normal- oder Ueberdruck, mit oder ohne Katalysator, durchgeführt. Geeignete Lösungsmittel sind z.B. Toluol, Xylol, Chlorbenzol, Dichlorbenzole, wie o-Dichlorbenzol, ferner Trichlorbenzole, Nitrobenzol oder Gemische aromatischer und/oder aliphatischer Lösungsmittel, wie ®Shellsole.

Die Isolierung der Verbindungen der Formel I erfolgt nach ihrer Synthese wie üblich z.B. durch Filtration. Das Nutschgut wird z.B. mit einem der bereits oben angegebenen Lösungsmittel und dann zweckmassig noch mit Wasser gewaschen. Sie werden im allgemeinen in guter Ausbeute und Reinheit erhalten und können im Prinzip selbst ohne weitere Reinigung in feinverteilter Form zum Massefärben von Kunststoffen, insbesondere von Polyolefinen, verwendet werden.

Sofern ihre Reinheit und/oder Partikel-Form und -Grösse zur Anwendung als Pigmente noch nicht genügend bzw. optimal sind, können die Verbindungen der Formel I weiter konditioniert werden. Unter Konditionierung versteht man die Herstellung einer feinen, für die Applikation optimalen Partikel-Form und -Grösse, beispielsweise durch Trockenmahlung mit oder ohne Salz, durch Lösungsmittel- oder wässrige Mahlung oder durch Salzknetung oder durch eine nachträgliche thermische Lösungsmittelbehandlung.Thermische Lösungsmittelbehandlungen können z.B. in organischen Lösungsmitteln, vorzugsweise mit solchen, die über 100 °C sieden, durchgeführt werden.

Als besonders geeignet hierfür erweisen sich durch Halogenatome, Alkyl- oder Nitrogruppen substituierte Benzole, wie Toluol, Chlorbenzol, o-Dichlorbenzol, Xylole oder Nitrobenzol, Alkohole, wie Isopropanol oder Isobutanol, ferner Ketone, wie Cyclohexanon, Ether, wie Ethylenglykol- monomethyl- oder -monoethylether, Amide, wie Dimethylformamid oder N-Methyl-pyrrolidon, sowie Dimethylsulfoxyd, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen oder aliphatischen Aminen, oder in flüssigem Ammoniak, durchführen.

Je nach Konditionierverfahren und/oder Applikationszweck kann es von Vorteil sein, der Verbindungen der Formel I gewisse Mengen an texturverbessernden Mitteln vor oder nach dem Konditionierprozess zuzufügen. Als solche kommen insbesondere Fettsäuren mit mindestens 18 C-Atomen, beispielsweise Stearin- oder Behensäure oder deren Amide oder Metallsalze, insbesondere Mg-Salze in Betracht. Die texturverbessernden Mittel werden vorzugsweise in Mengen von 0,1-30 Gew.%, insbesondere 2-15 Gew.%, bezogen auf das Endprodukt, zugesetzt.

Die erhaltenen Verbindungen der Formel I können aber aufgrund ihrer ausgezeichneten Polyolefinverträglichkeit oft ohne weitere Konditionierung, wie mechanische Zerkleinerung oder Präparierung, direkt in Polyolefine zu deren Färbung eingearbeitet werden.

Obwohl sie insbesondere zum Massefärben von Polyolefinen geeignet sind, können sie auch vorteilhaft zum Massefärben von anderen Polymeren, z.B. Polyvinylchlorid, Fluorpolymerisate, wie z.B. Polyfluorethylen, Polytrifluorchlorethylen oder Tetrafluorethylet/Hexafluorpropylen-Mischpolymerisat, insbesondere aber von Ingenieurwerkstoffen (Engineering Plastics), wie z.B. Polycarbonate, Polyacrylate, Polymethacrylate, ABS, Polyester, Polyamide, Polyetherketone, Polyurethane, einzeln oder in Mischungen, verwendet werden. Zweckmässig werden sie in einer Konzentration von 0,01 bis 5 Gew.%, bezogen auf das Polymere, eingesetzt.

Als Beispiele für Polyolefine, die mit den Verbindungen der Formel I gefärbt werden können, seien Polyethylen hoher und niederer Dichte (HD-PE, LD-PE und LLD-PE), Polypropylen und Polyisobutylen, sowie Copolymere von Polyolefinen mit z.B. Polyethern, Polyetherketonen oder Polyurethanen, erwähnt. Bevorzugt ist Polypropylen.

Die Färbung erfolgt nach den üblichen Verfahren, beispielsweise durch Mischen einer Verbindung der Formel I oder eines Gemisches solcher Verbindungen mit dem Kunststoffgranulat oder -pulver, ohne es

vorher in ein Präparat einarbeiten zu müssen, und Extrudieren der Mischung zu Fasern, Folien oder Granulaten. Letztere können dann im Spritzgussverfahren zu Gegenständen verformt werden.

Die erhaltenen Ausfärbungen weisen hohe Reinheit und hohe Sättigung auf und zeichnen sich durch gute Transparenz sowie durch gute Beständigkeit, insbesondere gegen Hitze und Licht aus. Ein besonderer Vorteil von mit den Verbindungen der Formel I gefärbten Polyethylengegenständen besteht darin, dass sie, insbesondere im Falle von HD-PE, keine erhöhte Neigung zu Verzugs- und Deformationserscheinungen zeigen.

Die mit den Verbindungen der Formel I gefärbten Fasern, insbesondere die Polyproplyenfasern sind brillant, besitzen ausgezeichnete textile Eigenschaften, wie z.B. Lichtbeständigkeit und Beständigkeiten gegenüber Wasch- und Lösungsmitteln. Der Einsatz von Verbindungen der Formel I ist auch mit einigen technischen Vorteilen verbunden, wie z.B. sehr leichte Dispergierbarkeit, weniger Faserbrüche, geringere Spinndüsenverstopfung und bessere Filterzeiten.

Die nachfolgenden Beispiele dienen zur Erläuterung der Erfindung.

Beispiel 1: Zu einer Suspension von 7,1 g 1,1'-Diamino-4,4'-dianthrachinon in 150 ml Chlorbenzol werden 14,6 g Stearoylchlorid zugetropft. Die Suspension wird auf 125 °C erhitzt, 18 Stunden bei dieser Temperatur gerührt, abkühlen gelassen, filtriert und das Nutschgut wird mit Methanol gewaschen. Der feuchte Farbkörper wird zur Reinigung zwei Stunden in Methanol gekocht und anschliessend filtriert. Nach nochmaligem Waschen mit Methanol und Trocknen im Vakuumtrockenschrank erhält man 15,0 g der Verbindung der Formel

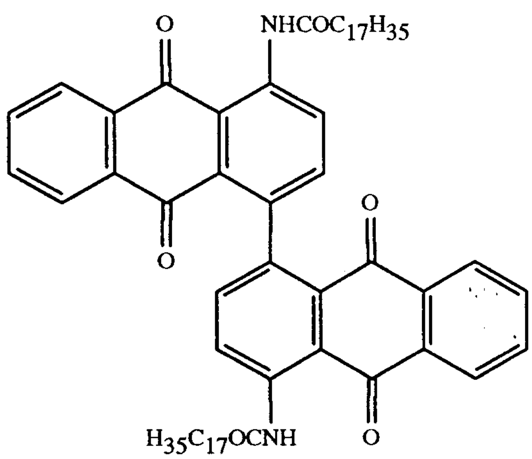

als grüngelbes Pulver.

| Analyse für $C_{64}H_{84}N_2O_6$ | | | |
|---|---|---|---|
| | C | H | N |
| Berechnet:<br>Gefunden: | 78,65<br>78,58 | 8,66<br>8,43 | 2,87<br>2,70 |

Beispiele 2 und 3: Verfährt man wie in Beispiel 1 mit der einzigen Ausnahme, dass man Stearoylchlorid mit der äquivalenten Menge eines Säurechlorids der Formel XCOCl, worin X die in nachfolgender Tabelle angegebene Bedeutung hat, so erhält man entsprechende Verbindungen der Formel I. Die Resultate sind in der nachfolgenden Tabelle zusammengefasst.

| Beispiel | X | Ausbeute | Farbe | Analyse (%) | C | H | N | Cl |
|---|---|---|---|---|---|---|---|---|
| 2 | $C_{11}H_{23}$- | 65 % | Gelb | Ber.:<br>Gef.: | 77,2<br>77,2 | 7,5<br>7,5 | 3,5<br>3,4 | -<br>- |
| 3 | $C_7H_{15}CH{=}CH{-}(CH_2)_7$- | 63 % | Gelb | Ber.:<br>Gef.: | 77,0<br>77,3 | 11,6<br>11,7 | 2,0<br>2,2 | -<br>- |
| 4 | $C_{15}H_{31}$- | 59 % | Gelb | Ber.:<br>Gef.: | 78,4<br>78,0 | 8,1<br>8,3 | 3,1<br>2,7 | -<br>- |
| 5 | 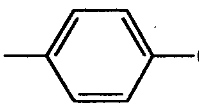 $-OC_{18}H_{37}$ | 89 % | Gelb | Ber.:<br>Gef.: | 78,8<br>78,8 | 8,1<br>8,1 | 2,4<br>2,4 | -<br>- |
| 6 | 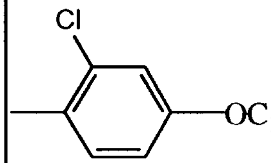 $-OC_{18}H_{37}$ | 87 % | Gelb | Ber.:<br>Gef.: | 74,4<br>74,2 | 7,5<br>7,7 | 2,2<br>2,1 | 5,6<br>5,7 |

Beispiel 7: Eine Mischung von 1,0 g des nach Beispiel 1 erhaltenen Pigmentes, 1,0 g Antioxidans (IRGANOX® 1010, CIBA-GEIGY AG) und 1000 g Polyethylen-HD Granulat (®VESTOLEN 60-16, HUELS) wird während 15 Minuten in einer Glasflasche auf einer Rollbank vorgemischt. Danach wird die Mischung in zwei Passagen auf einem Einwellenextruder extrudiert, das so erhaltene Granulat wird auf der Spritzgussmaschine (Allround Aarburg 200) bei 220 °C zu Platten verspritzt und 5 Minuten bei 180 °C nachgepresst. Die Pressplatten weisen farbstarke gelbe Nuancen mit guten Beständigkeiten auf.

Beispiel 8: 0,6 g des gemäss Beispiel 1 erhaltenen Pigmentes werden mit 67 g Polyvinylchlorid, 33 g Dioctylphthalat, 2 g Dibutylzinndilaurat und 2 g Titandioxid vermischt und auf einem Walzenstuhl während 15 Minuten bei 160 °C zu einer dünnen Folie verarbeitet. Die so erzeugte gelbe PVC-Folie ist farbstark und lichtbeständig.

Beispiel 9: 400 g Polypropylengranulat (®DAPLEN PT-55, Chemie LINZ) und 4 g des nach Beispiel 1 erhaltenen Pigmentes werden in einer Mischtrommel intensiv vermischt. Das so behandelte Granulat wird bei 260 bis 285 °C nach dem Schmelzspinnverfahren versponnen. Man erhält gelb gefärbte Fasern mit sehr guten textilen Eigenschaften, wie Licht- und Nassbeständigkeiten.

## Patentansprüche

1. Kunststoffe enthaltend in der Masse Dianthrachinonylverbindungen der Formel

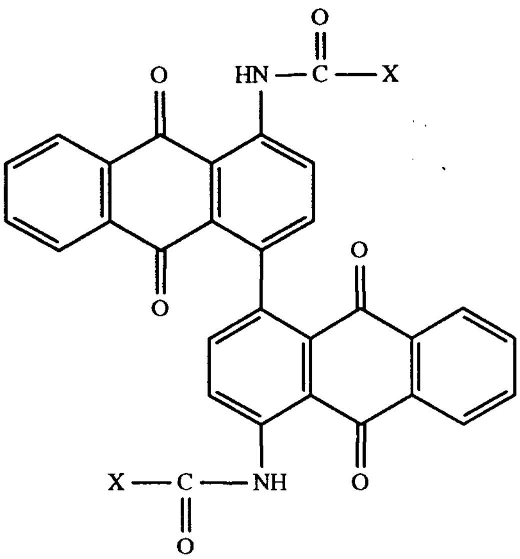

(I),

worin X einen Rest $R_1$, $R_2$, $R_3$ oder $R_4$ bedeutet, wobei
$R_1$ Alkyl oder Alkenyl mit mindestens 8 C-Atomen,
$R_2$ $-OR_1$ oder $-SR_1$,
$R_3$ $-NHR_1$ oder $-N(R_1)_2$ sind und
$R_4$ eine Gruppe der Formel

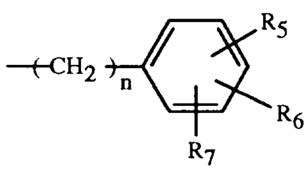

bedeutet, worin
$R_5$ $-NHR_1$, $-N(R_1)_2$, $-OR_1$ oder $-SR_1$ ist und n Null, 1 oder 2 bedeutet und $R_6$ und $R_7$, unabhängig voneinander, Wasserstoff, Halogen, Nitro, $C_1$-$C_4$-Alkyl oder $C_1$-$C_4$-Alkoxy bedeuten.

2. Kunststoffe gemäss Anspruch 1, dadurch gekennzeichnet, dass sie Dianthrachinonylverbindungen der Formel I, worin $R_1$ $C_{10}$-$C_{35}$-Alkyl bedeutet, enthalten.

3. Kunststoffe gemäss Anspruch 1, dadurch gekennzeichnet, dass sie Dianthrachinonylverbindungen der Formel I, worin $R_1$ $C_{12}$-$C_{18}$-Alkyl bedeutet, enthalten.

4. Kunststoffe gemäss Anspruch 1, dadurch gekennzeichnet, dass sie Dianthrachinonylverbindungen der Formel I enthalten, worin X $C_{12}$-$C_{18}$-Alkyl oder eine Gruppe der Formel

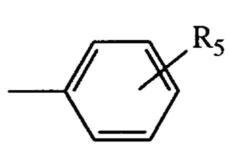

bedeutet,
worin $R_5$ $-NHR_1$, $-N(R_1)_2$, $-OR_1$ oder $-SR_1$ ist und $R_1$ $C_{12}$-$C_{18}$-Alkyl bedeutet.

5. Kunststoffe gemäss Anspruch 1, dadurch gekennzeichnet, dass sie Dianthrachinonylverbindungen der Formel I, worin X geradkettiges $C_{12}$-$C_{18}$-Alkyl bedeutet enthalten.

6. Kunststoffe gemäss Anspruch 1, dadurch gekennzeichnet, dass es sich um Polyolefine handelt.

7. Kunststoffe gemäss Anspruch 1, dadurch gekennzeichnet, dass es sich um Polypropylen handelt.

**Claims**

1. A plastics material comprising, in the mass, dianthraquinonyl compounds of the formula

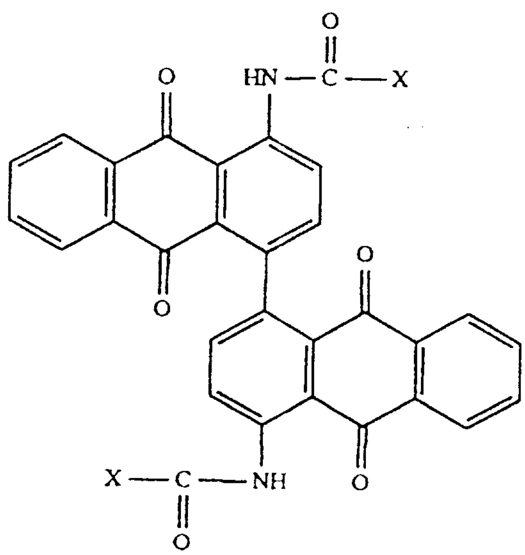

(I),

wherein X is a radical $R_1$, $R_2$, $R_3$ or $R_4$, and
$R_1$ is alkyl or alkenyl of not less than 8 carbon atoms,
$R_2$ is $-OR_1$ or $-SR_1$,
$R_3$ is $-NHR_1$ or $-N(R_1)_2$, and
$R_4$ is a group of the formula

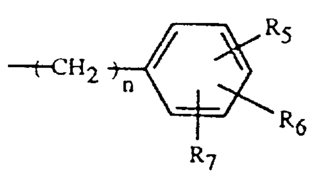

wherein
$R_5$ is $-NHR_1$, $-N(R_1)_2$, $-OR_1$ or $-SR_1$ and n is zero, 1 or 2, and
$R_6$ and $R_7$ are each independently of the other hydrogen, halogen, nitro, $C_1$-$C_4$alkyl or $C_1$-$C_4$alkoxy.

2. A plastics material according to claim 1, which contains dianthraquinonyl compounds of the formula I, wherein $R_1$ is $C_{10}$-$C_{35}$alkyl.

3. A plastics material according to claim 1, which contains dianthraquinonyl compounds of the formula I, wherein $R_1$ is $C_{12}$-$C_{18}$alkyl.

4. A plastics material according to claim 1, which contains dianthraquinonyl compounds of the formula I, wherein X is $C_{12}$-$C_{18}$alkyl or a group of the formula

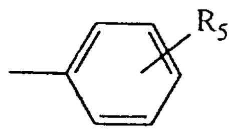

wherein $R_5$ is $-NHR_1$, $-N(R_1)_2$, $-OR_1$ or $-SR_1$ and $R_1$ is $C_{12}$-$C_{18}$alkyl.

**5.** A plastics material according to claim 1, which contains dianthraquinonyl compounds of the formula I, wherein X is straight-chain $C_{12}$-$C_{18}$alkyl.

**6.** A plastics material according to claim 1, which is a polyolefin.

**7.** A plastics material according to claim 1, which is polypropylene.

**Revendications**

**1.** Matières plastiques contenant dans la masse des composés de dianthraquinonyle de formule :

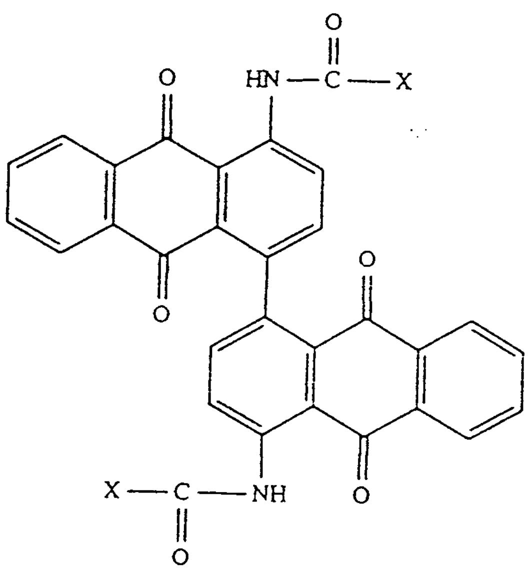

(I)

dans laquelle X signifie un radical $R_1$, $R_2$, $R_3$ ou $R_4$,

$R_1$ signifiant un alkyle ou un alcényle comportant au moins 8 atomes de carbone,
$R_2$ signifiant $-OR_1$ ou $-SR_1$,
$R_3$ signifiant $-NHR_1$ ou $-N(R_1)_2$ et
$R_4$ signifiant un groupe de formule

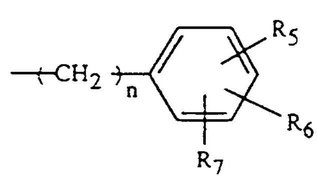

dans laquelle

$R_5$ représente $-NHR_1$, $-N(R_1)_2$, $-OR_1$ ou $-SR_1$ et n vaut 0, 1 ou 2 et
$R_6$ et $R_7$, indépendamment l'un de l'autre, représentent l'hydrogène, les halogène, nitro, alkyle en $C_1$-$C_4$ ou alcoxy en $C_1$-$C_4$.

**2.** Matières plastiques selon la revendication 1, caractérisées en ce qu'elles contiennent des composés de dianthraquinonyle de formule I, dans laquelle $R_1$ représente un alkyle en $C_{10}$-$C_{35}$.

**3.** Matières plastiques selon la revendication 1, caractérisées en ce qu'elles contiennent des composés de dianthraquinonyle de formule I, dans laquelle $R_1$ représente un alkyle en $C_{12}$-$C_{18}$.

**4.** Matières plastiques selon la revendication 1, caractérisées en ce qu'elles contiennent des composés de dianthraquinonyle de formule I, dans laquelle X représente un alkyle en $C_{12}$-$C_{18}$ ou un groupe de formule

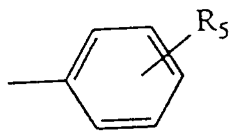

dans laquelle $R_5$ représente $-NHR_1$, $-N(R_1)_2$, $-OR_1$ ou $-SR_1$ et $R_1$ signifie un alkyle en $C_{12}$-$C_{18}$.

**5.** Matières plastiques selon la revendication 1, caractérisées en ce qu'elles contiennent des composés de dianthraquinonyle de formule I, dans laquelle X représente un alkyle en $C_{12}$-$C_{18}$ linéaire.

**6.** Matières plastiques selon la revendication 1, caractérisées en ce qu'il s'agit de polyoléfines.

**7.** Matières plastiques selon la revendication 1, caractérisées en ce qu'il s'agit du polypropylène.